# EUROPÄISCHE PATENTSCHRIFT

(11) **EP 1 482 875 B1**
(45) Veröffentlichungstag und Bekanntmachung des Hinweises auf die Patenterteilung: **11.03.2009**
(21) Anmeldenummer: 02782913.4
(22) Anmeldetag: 15.10.2002
(51) Int. Cl.: A61F 2/44

(54) **ZWISCHENWIRBELPROTHESE**
INTRAVERTEBRAL PROSTHESIS
PROTHESE INTERVERTEBRALE

(30) Priorität: 12.03.2002 EP 02005632; 12.03.2002 EP 02005631; 12.03.2002 EP 02005630
(43) Veröffentlichungstag der Anmeldung: 08.12.2004
(73) Patentinhaber: Cervitech Inc., Rockway, NJ 07866 (US)
(72) Erfinder: KELLER, Arnold, 23863 Kayhude (DE); MCAFEE, Paul C., c/o Scoliosis and Spine Center, Baltimore, MD 21204 (US)
(74) Vertreter: Glawe, Delfs, Moll
(86) Internationale Anmeldenummer: PCT/EP2002/011524
(87) Internationale Veröffentlichungsnummer: WO 2003/075803

(56) Entgegenhaltungen:
- EP-A- 0 978 258
- WO-A-01/01893
- WO-A-99/65412
- FR-A- 2 718 635
- US-A- 5 425 773

## Beschreibung

Zwischenwirbelprothesen dienen dem Ersatz der Bandscheibe. Sie bestehen aus zwei Deckplatten, deren Außenflächen zur Verbindung mit benachbarten Wirbelkörpern ausgebildet sind, und einer von den Deckplatten eingeschlossenen Gelenkeinrichtung. Bei einem bekannten Prothesentyp (WO 01/01893, FR-A-2718635) bildet die obere Deckplatte eine konkav-sphärische Gelenkfläche auf ihrer Innenseite, die mit der konvexsphärischen Oberseite eines aus Polyethylen bestehenden Prothesenkerns zur Bildung eines Gelenks zusammenwirkt. Die flache Unterseite und der Rand des Kerns sind passend in einem Sitz aufgenommen, der von der unteren Deckplatte gebildet ist. Dieser Sitz besteht aus einer ebenen Bodenfläche und einem diese an drei Seiten (seitlich und dorsal) umgebenden, hochstehenden Rand. An den Seiten wird der Rand von zwei sich im wesentlichen in AP-Richtung erstreckenden, hinterschnittenen Randleisten gebildet, denen komplementär vorspringende Leisten bzw. Nuten am Rand des Prothesenkerns entsprechen. An der ventralen Seite ist der Rand der Deckplatte offen, so daß der Prothesenkern schubladenartig in einer in AP-Richtung verlaufenden Bewegung in den Rand der Deckplatte eingeschoben werden kann. Im eingeschobenen Zustand ist der Prothesenkern durch die zusammenwirkenden vorspringenden Leisten und Nuten der Deckplatte und des Prothesenkerns gegen Abheben gesichert. Insbesondere kann er sich aus der vorgesehenen Position nicht dorsal zum Rückenmark hin entfernen wenn die Deckplatten sich bei einer Beugebewegung dorsal aufspreizen.

Es gibt bekannte Zwischenwirbelprothesen, bei denen der Rand der unteren Deckplatte auch ventral geschlossen ist (EP-B-471821, US-A-5425773). Jedoch hat dies den Nachteil, daß der Prothesenkern entweder nach dem Einsetzen der Deckplatten nicht zwischen diese eingefügt werden kann oder im Sitz der unteren Deckplatte nicht gegen Abheben gesichert ist. Die Erfindung bezieht sich dagegen ausschließlich auf denjenigen Prothesentyp, bei welchem der Sitz der unteren Deckplatte als ventral offene Einschubführung für den Prothesenkern ausgebildet ist, damit er nach der Implantation der Deckplatten in die Prothese eingefügt werden kann.

Damit der Prothesenkern nicht ventral aus dem Sitz entweichen kann, ist bei dem bekannten Prothesen dieser Art ein Rastanschlag vorgesehen (FR-A-2 718 635, WO 01/01893). Dieser besteht aus zusammenwirkenden Vorsprüngen und Ausnehmungen in der Unterseite des Prothesenkerns einerseits und der Bodenfläche des Sitzes andererseits. Damit diese beim Einschieben des Prothesenkerns in den Sitz in Eingriff miteinander gelangen können, muß der aus nachgiebigen Kunststoffmaterial bestehende Sitz sich vor dem Verrasten dieser Elemente elastisch verformen. Dies hat den Nachteil, daß die Verrastung prinzipiell unsicher ist, weil sie durch eine entsprechende elastische Verformung des Prothesenkerns wieder gelöst werden kann. Zwar kann man die Rastsicherheit erhöhen, indem man dem Prothesenkern einen größtmöglichen Verformungswiderstand abverlangt. Aber dadurch wird dem Operateur das Einschieben des Prothesenkerns in den Sitz erschwert. Auch kann es geschehen, daß die Rastelemente aus zufälligen Gründen, die dem Operateur verborgen bleiben, die Raststellung nicht oder nicht vollständig erreichen, weil z.B. in der Rastausnehmung ein Fremdkörper verblieben ist oder weil der Prothesenkern wegen eines zufälligen Hindernisses nicht weit genug in den Sitz hineingeschoben wurde.

Der Erfindung liegt somit die Aufgabe zugrunde, eine Zwischenwirbelprothese des geschilderten Typs zu schaffen, bei welcher der Prothesenkern sicher in der Prothese gehalten ist und die Operation nicht erschwert wird.

Die erfindungsgemäße Lösung findet sich in der Merkmalskombination des Anspruchs 1.

Um dem Prothesenkern die AP-Bewegung zu gestatten, die für das Einschieben in den Sitz erforderlich ist, ist der Sitz als Führungseinrichtung mit einer in AP-Richtung verlaufenden Richtung ausgebildet. Diese kann von einander gegenüberliegenden, parallelen, seitlichen Führungsschienen gebildet sein, zwischen denen der Kern so gehalten ist, daß er sich lediglich in AP-Richtung bewegen kann. Dabei sind die Führungsschienen zweckmäßigerweise hinterschnitten, um mit einer in den Hinterschnitt eingreifenden Leiste des Kerns zusammenzuwirken. Auf diese weise wird verhindert, daß der Kern sich von der ihn haltenden Deckplatte abhebt. Dies hat den Vorteil, daß die für die Eingrenzung der Kernbewegung vorgesehenen Einrichtungen nicht sehr hoch sein müssen und daher auch keine Gefahr besteht, daß sie die Relativbewegung der Deckplatten im Verhältnis zueinander behindern könnten. Damit der Kern nicht dorsal oder ventral aus dem Führungsbereich der Schienen herausgleitet, sind entsprechende Anschläge vorgesehen. Der dorsale Bewegungsanschlag ist zweckmäßigerweise starr mit der den Sitz (d.h. die Führungsschienen) bildenden Deckplatte verbunden. Auf der ventralen Seite ist ein Bewegungsanschlag vorgesehen, der aus seiner sperrenden Stellung entfernbar ist, damit man den Kern nach der Implantation der Deckplatte leichter einsetzen kann. Anschließend wird der Anschlag in der Stellung fixiert, in der er das Entweichen des Kerns verhindert.

Am ventralen Rand der den Sitz bildenden Deckplatte ist eine Gleitführung vorgesehen, in oder an welcher der Anschlag zwischen einer sperrenden Stellung und einer nicht sperrenden Stellung verschiebbar ist. Dadurch ist die Voraussetzung dafür geschaffen, daß der Operateur den Anschlag bewußt in die sperrende Stellung verbringen muß. Die sperrende Stellung des Anschlags wird daher mit Sicherheit erreicht.

Besonders zweckmäßig ist eine Ausführungsform, bei welcher die Gleitführung von einem quer zur Führungseinrichtung verlaufenden Führungsschlitz gebildet ist, der den als Anschlagplatte ausgebildeten Anschlag aufnimmt.

Beispielsweise kann der Führungsschlitz eine quer zur Erstreckungsebene der Deckplatte verlaufende Führungsrichtung haben. D.h. daß die Anschlagplatte in ihrer sperrenden Stellung zu einem Teil in dem Schlitz steckt und von diesem gehalten wird und zu einem anderen Teil über die Bodenfläche des Sitzes hinausragt, um den Prothesenkern in dem Sitz festzuhalten. In der nicht sperrenden Stellung ist sie unter die Bodenfläche des Sitzes in den Schlitz abgesenkt oder aus diesem gänzlich nach unten oder oben entfernt.

Statt dessen kann der Führungsschlitz auch eine parallel zur Erstreckungsebene der ersten Deckplatte und quer zur AP-Richtung verlaufende Führungsrichtung haben. In diesem Fall wird die Anschlagplatte von der Seite her in den Führungsschlitz eingeschoben, um in die sperrende Stellung zu gelangen.

In jedem Fall sollten Sicherungseinrichtungen vorhanden sein, die die Anschlagplatte in der sperrenden Stellung festhalten. Eine besonders zuverlässige Sicherung besteht darin, daß die Anschlagplatte von einer Befestigungsschraube durchquert ist, so daß sich die Anschlagplatte aus der Sicherungsstellung nur dann entfernen kann, wenn zuvor die Schraube entfernt wurde. Zu diesem Zweck wird der Führungsschlitz von wenigstens einem Schraubenloch durchquert und weist die Anschlagplatte ein in ihrer sperrenden Stellung damit fluchtendes Schraubenloch auf. Diese Ausführung ist insbesondere dann vorteilhaft, wenn der Führungsschlitz in einem Befestigungsflansch liegt, der am ventralen Rand der den Sitz bildenden Deckplatte vorgesehen ist.

Eine weitere Sicherung, durch die die Anschlagplatte gegen ein Entweichen aus dem Führungsschlitz gesichert wird, besteht darin, daß der Prothesenkern einen Teil aufweist, der die im Führungsschlitz enthaltene Anschlagplatte überdeckt.
Diese Überdeckung kann als ein Schlitz im Prothesenkern ausgebildet sein, der mit dem Führungsschlitz fluchtet, wenn der Prothesenkern die vorgesehene Stellung im Sitz erreicht hat. Der Führungsschlitz in der Deckplatte einerseits und der damit fluchtende Schlitz im Prothesenkern andererseits bilden dann eine Aufnahme für die Anschlagplatte, in die sie leicht von der Seite eingeschoben werden kann.

Eine weitere Sicherungsmöglichkeit besteht darin, daß die Anschlagplatte eine in der sperrenden Stellung in der Richtung des Führungsschlitzes über den Führungsschlitz hinausragende, leicht umbiegbare Sicherungsnase aufweist. Diese wird vom Operateur nach dem Einsetzen so verbogen, daß sie aus der Ebene der Anschlagplatte herausragt und dadurch die Rückbewegung im Führungsschlitz unmöglich macht.

Bei einer anderen erfindungsgemäßen Ausführung der Anschlagplatte wird diese nicht translatorisch aus der nicht sperrenden Stellung in die sperrende Stellung überführt, sondern durch eine Schwenkbewegung. In diesem Falle wird die Gleitführung, die die Anschlagplatte aufnimmt von einer am ventralen Rand der Deckplatte quer zur AP-Richtung verlaufenden Gleitführungsfläche und einem sich davon erhebenden Schwenkbolzen gebildet, auf dem die Anschlagplatte schwenkbar gelagert ist. Die Anschlagplatte weist eine Zunge auf, die in einer sperrenden Schwenkstellung der Anschlagplatte über die Bodenfläche des Sitzes hinausragt und damit das Entweichen des Prothesenkerns aus dem Sitz hindert. In der nicht sperrenden Stellung ist diese Anschlagzunge unter die Bodenfläche des Sitzes abgesenkt. Die Gleitführungsfläche, an der die Anschlagplatte gehalten ist, wird zweckmäßigerweise von der Stirnfläche eines Befestigungsflanschs gebildet. Dieser enthält auch mindestens ein Schraubenloch in der Nachbarschaft des Schwenkbolzens. Die Anschlagplatte kann erfindungsgemäß zur Sicherung der in diesem Schraubenloch befindlichen Schraube genutzt werden, indem sie mit einem Flügel über den in dem Schraubenloch befindlichen Schraubenkopf greift.

Der erfindungsgemäße Anschlag ist nicht nur für diejenigen Fälle geeignet, in denen der Prothesenkern eine feste unbewegliche Stellung im Sitz einnimmt, sondern auch für diejenigen Fälle, in denen ihm Bewegungsfreiheit, insbesondere in AP-Richtung gegeben ist. Die von dem Sitz gebildete Führungseinrichtung für den Prothesenkern wird dann nicht nur für dessen Einschubbewegung genutzt sondern auch für eine ständige Beweglichkeit. Diese kann vorteilhaft sein, insbesondere bei Zervikalprothesen, bei denen die Gelenkfläche des Prothesenkerns sich über im wesentlichen die gesamte Deckplatte erstreckt. In diesen Fällen möchte man nämlich den Krümmungsradius der Gelenkfläche gering halten, um die Bauhöhe der Prothese gering zu halten. In solchen Fällen kann eine AP-Beweglichkeit des Prothesenkerns die Annäherung der Beugeeigenschaften der Prothese an die natürlichen Verhältnisse verbessern. Ein besonderer Aspekt des Erfindungsgedankens besteht deshalb darin, daß innerhalb eines Systems von Zwischenwirbelprothesen neben solchen, die diese AP-Beweglichkeit aufweisen, andere Prothesen mit vorzugsweise übereinstimmender Außenform vorhanden sind, die keine AP-Beweglichkeit zwischen dem Prothesenkern und der ihn haltenden Deckplatte zeigen. Dies ermöglicht es dem Arzt, intraoperativ zu entscheiden, ob er AP-Beweglichkeit vorsehen will oder nicht. Zweckmäßigerweise sind die Deckplatten der in AP-Richtung beweglichen bzw. unbeweglichen Prothesen übereinstimmend geformt und ist lediglich der Kern unterschiedlich. Es kann aber auch vorgesehen sein, daß der Prothesenkern und die mit ihm das Gelenk bildende Deckplatte in allen Typen übereinstimmen, während die AP-Beweglichkeit durch Unterschiedlichkeit der den Prothesenkern haltenden Deckplatte begründet ist. Schließlich besteht auch die Möglichkeit, daß alle drei Komponenten übereinstimmen und lediglich derjenige Anschlag, der ventral den Weg des Prothesenkerns in AP-Richtung begrenzt, unterschiedlich lokalisiert ist.

Wenn hier von unterer und oberer Deckplatte die Rede ist, so soll damit nicht Ausdruck gebracht werden, daß die den Sitz für den Kern bildende Deckplatte stets unten angebracht sein müßte. Vielmehr kann die Anordnung auch umgekehrt gewählt werden. In den Ansprüchen wird deshalb allgemeiner von einer ersten und einer zweiten Deckplatte gesprochen.

Bevorzugte Ausführungsformen werden im folgenden anhand der Zeichnungen erläutert. Darin zeigen:
- Fig. 1: einen Frontalschnitt
- Fig. 2: einen Sagittalschnitt und
- Fig. 3: eine Zerlegungsdarstellung einer ersten Ausführungsform;
- Fig. 4: eine Variante der ersten Ausführungsform;
- Fig. 5: einen Sagittalschnitt und
- Fig. 6: eine Zerlegungsdarstellung einer zweiten Ausführungsform;
- Fig. 7: die zweite Ausführungsform mit umgestellter Anschlagplatte;
- Fig. 8 und 9: zwei Sagittalschnitte gem. den Schnittlinien B bzw. C in Fig. 10;
- Fig. 10 und 11: zwei perspektivische Darstellungen der dritten Ausführungsform und
- Fig. 12: die Anschlagplatte der dritten Ausführungsform;
- Fig. 13: eine Zerlegungsdarstellung einer vierten Ausführungsform und
- Fig. 14: eine Variante der unteren Deckplatte der vierten Ausführungsform.

Die untere Deckplatte 1 und die obere Deckplatte 2 der ersten Ausführungsform weisen äußere Oberflächen 3 bzw. 4 auf, die zur Verankerung am zugehörigen Wirbelkörper bestimmt sind. Sie sind vorzugsweise eben. Es sind aber auch andere im wesentlichen flache Gestaltungen einschließlich geeigneter Oberflächenstrukturen zur besseren Verankerung am Knochen denkbar. Die Deckplatten bestehen vorzugsweise aus Metall.

Die untere Deckplatte 1 wendet der oberen Deckplatte 2 eine ebene Bodenfläche 5 zu, die auf drei Seiten von einem Kragen 6 eingefaßt ist, der oberhalb eines inneren Hinterschnitts eine nach innen ragende Leiste 7 bildet. Die untere Deckplatte 1 ist in der Draufsicht oval oder etwa rechteckig gestaltet.

Die Bodenfläche 5 und der Kragen 6 der unteren Deckplatte 1 bilden einen Sitz für den Prothesenkern 10, der aus gleitgünstigem Werkstoff, beispielsweise Polyethylen, besteht. Er hat eine zur Bodenfläche 5 passende ebene Unterfläche, die seitlich und dorsal von einer Randleiste 11 umgrenzt wird., oberhalb welcher sich eine Nut 12 befindet. Die Leiste 11 greift in den Hinterschnitt des Kragens 6 unterhalb der Leiste 7. Die Leiste 7 greift in die Nut 12 ein. Dadurch ist der Prothesenkern 10 gegen Abheben von der unteren Deckplatte 1 gesichert. Zwischen dem Kragen 6 und der unteren Deckplatte 1 und dem Rand des Kerns 10 ist Gleitspiel vorhanden. Im Bereich ihrer Seiten 8, 9 verlaufen die dortigen Zweige des Kragens 6 bzw. der Leisten 7 und 11 und der Nut 12 zueinander parallel und gradlinig.

Der Kern 10 hat allseits im wesentlichen dieselbe Umrißgestalt in der Draufsicht wie die untere Deckplatte 1 und die obere Deckplatte 2. Er überdeckt insbesondere den Kragen 6, so daß die Größe der von der Oberseite des Kerns zur Verfügung gestellten Gleitfläche 22 durch das Vorhandensein des Kragens 6 nicht verringert wird. Der Kragen 6 kann im Verhältnis zur Höhe des Kerns 10 klein ausgebildet sein. Trotzdem kann der Kern dem Zwischenraum zwischen den Deckplatten 1 und 2 nicht entweichen, weil er durch das Zusammenspiel der hinterschnittenen Leisten 7 und 11 am Abheben von der Deckplatte 1 gehindert wird.

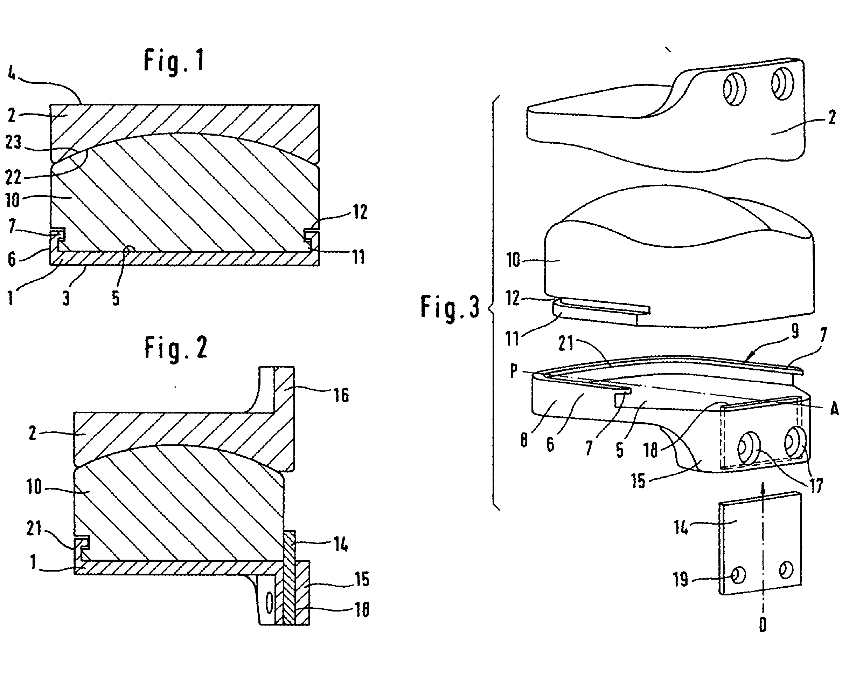

An ihrem ventralen Rand weisen die Deckplatten 1, 2 je einen etwa rechtwinklig von ihnen abragenden Befestigungsflansch 15, 16 auf, der Schraubenlöcher 17 zur Befestigung am Wirbelkörper enthält. Im Flansch 15 der unteren Deckplatte 1 befindet sich ein Schlitz 18, der eine Gleitführung für eine Anschlagplatte 14 bildet, die darin verschiebbar gehalten ist. Sie kann die in Fig. 2 dargestellte Sperrstellung einnehmen, in welcher sie einen Anschlag für die nach vorne gerichtete Bewegung des Kerns 10 bildet. Sie kann in den Schlitz 18 auch soweit nach unten verschoben oder gänzlich daraus entfernt werden, daß der Prothesenkern leicht von ventral in den Sitz der unteren Deckplatte und zwischen die Deckplatten eingebracht werden kann. Sie enthält zwei Bohrungen 19, die in der Sperrstellung mit den Schraubenlöchern 17 fluchten. Wenn die untere Deckplatte 1 durch die Schraubenlöcher 17 hindurch am Wirbelkörper befestigt ist, gehen die Befestigungsschrauben auch durch die Löcher 19 und sichern dadurch die Anschlagplatte 14 in ihrer Sperrstellung.

Die seitlichen Zweige des Kragens 6 bilden mit ihrer Leiste 7 im Zusammenwirken mit der Leiste 11 und der Nut 12 des Kerns 10 eine Führungseinrichtung für den Prothesenkern 10, in welche dieser von der offenen ventralen Seite (in Fig. 2 rechts) her in AP-Richtung (in Fig. 3 angedeutet) eingeschoben werden kann. Der dorsale Teil 21 des Kragens 6 wirkt als Sicherungsanschlag, der ein Entweichen des Kerns in dorsaler Richtung aus dem Zwischenraum zwischen den Deckplatten 1 und 2 verhindert. Auf das Vorhandensein des Hinterschnitts an dem Kragen 6 und am Rand des Kerns 10 kommt es für die Führungsfunktion nur in den seitlichen Bereichen 8 und 9 der unteren Deckplatte 1 und des Kerns 10 an, nicht aber im dorsalen Verlauf 21 des Kragens 6.

Oberseitig weist der Kern 10 eine vorzugsweise konvexsphärische Gelenkfläche 22 auf, die zur Bildung eines Gelenks zusammenwirkt mit der unterseitigen , konkav-sphärischen Gleitfläche 23 der oberen Deckplatte 2.

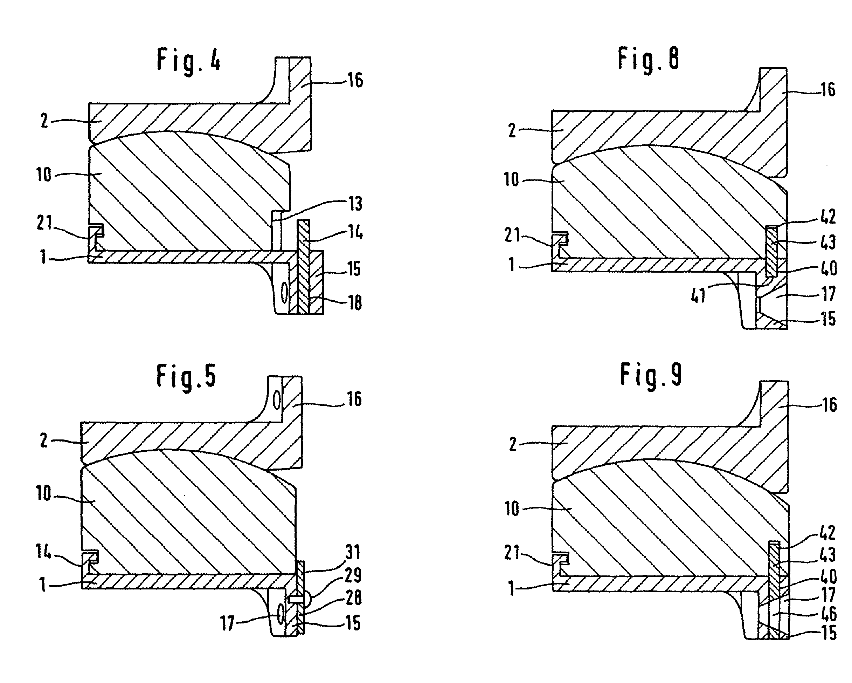

Während der Prothesenkern 10 in der Ausführungsform gemäß Fig. 2 in montiertem Zustand unbeweglich festgelegt ist zwischen dem dorsalen Anschlag 21 und dem ventralen Anschlag 14, zeigt Fig. 4 eine Variante, in welcher der Prothesenkern 10 ventral (in Fig. 4 rechts) ein wenig kürzer ist als die untere Deckplatte, so daß zwischen ihrer ventralen Endfläche 13 und de Anschlag 14 Spiel verbleibt, wenn sich der Prothesenkern in seiner weitest dorsalen Stellung befindet. Um die Größe dieses Spiels kann sich der Kern 10 im montierten Zustand in der AP-Richtung. Bei der Beugebewegung schwenkt die obere Deckplatte 2 im Verhältnis zur unteren Deckplatte 1 in der Ansicht gemäß Fig. 4 ein wenig im Uhrzeigersinn und bei der Streckbewegung entgegengesetzt. Wenn die obere Deckplatte 2 dabei genau der durch die Gleitflächen 22, 23 vorgegebenen Richtung folgt, ist diese Schwenkbewegung mit einer translatorischen Bewegung verknüpft, die bei der Beugung nach vorne (in Fig. 4 nach rechts) und bei der Streckung nach hinten (in Fig. 4 nach links) gerichtet ist. Ein Teil dieser translatorischen Bewegung kann den physiologischen Gegebenheiten widersprechen und zu unerwünschten Spannungen führen. Diese Spannungen verursachen rückstellende Kräfte, die bei der erfindungsgemäßen Prothesengestaltung dazu führen, daß die obere Deckplatte sich relativ zur unteren Deckplatte entgegengesetzt bewegt und dadurch die unerwünschte Bewegungskomponente kompensiert.

Zwischen den zusammenwirkenden Führungseinrichtungen des Kerns und der Deckplatte kann in seitlicher Richtung so viel Spiel gelassen werden, daß auch in dieser Richtung eine gewisse Relativbewegung möglich ist.

Das Ausmaß des Bewegungsspiels in AP-Richtung liegt bei einer Zervikalprothese vorzugsweise zwischen ein und vier, weiter vorzugsweise in der Größenordnung von zwei bis drei Millimetern. Falls eine Relativbeweglichkeit in seitlicher Richtung vorgesehen ist, sollte deren Ausmaß nicht über zwei Millimeter hinausgehen.

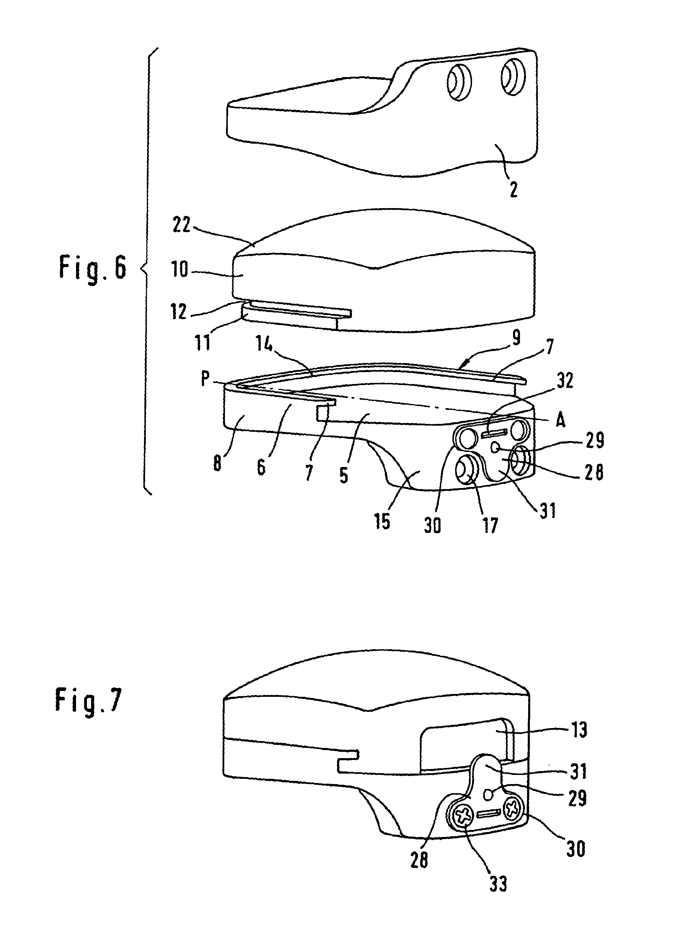

Für das zweite Ausführungsbeispiel gilt die obige Beschreibung mit Ausnahme des ventralen Anschlags. An ihrem ventralen Rand weisen die Deckplatten 1, 2 je einen rechtwinklig von ihnen abragenden Flansch 15, 16 auf, der Schraubenlöcher 17 zur Befestigung am Wirbelkörper enthält. Am Flansch 15 der unteren Deckplatte 1 ist mittig zwischen zwei Schraubenlöchern 17 eine Anschlagplatte 28 mittels eines Kopfbolzens 29 drehbar befestigt. Die Ebene, in der sie sich gleitend drehen kann, wird durch die Frontfläche des Befestigungsflanschs 15 bestimmt, die deshalb als Gleitführungsfläche bezeichnet wird. Die Anschlagplatte weist zwei sich im wesentlichen zur Seite hin erstreckende Flügel 30 und eine sich quer dazu erstreckende Zunge 31 auf. Sie besteht aus federndem Metall und ist so vorgespannt, daß ihre Flügel 30 gegen die Gleitführungsfläche drücken. Zum Eingriff eines Drehwerkzeugs, beispielsweise eines Schraubenziehers, enthält sie eine passende Öffnung oder Vertiefung 32. Wenn sie sich in der in Fig. 6 dargestellten Montagestellung befindet, ragt sie nicht über die Bodenfläche 5 der unteren Deckplatte hinaus. Der Kern 10 kann daher unbehindert in die von dem Kragen 6 gebildete Einschubführung eingeschoben werden. Wird sie um 180° gedreht, wie es in Fig. 5 und 7 dargestellt ist, ragt die Zunge 31 über die Oberfläche 5 und bildet dadurch einen Anschlag, der den Kern 10 am Verlassen der Führung zur ventralen Seite hin hindert.

In der in Fig. 6 gezeigten Montagestellung läßt die Anschlagplatte 28 die Schraubenlöcher 17 für die Montage der Befestigungsschrauben frei. Wie in Fig. 7 gezeigt, überdecken die Flügel 30 in der Sicherungsstellung ganz oder teilweise die Schraubenlöcher 17 und drücken elastisch auf die darin befindlichen Schraubenköpfe, um sie am Heraustreten aus den Schraubenlöchern 17 zu hindern. Sie enthalten eine Öffnung, die sich unter der Vorspannung einrastend über die Kuppen der in Fig 7 angedeuteten Schraubenköpfe 33 legt und dadurch die Sicherungsplatte am Verlassen der Sicherungsstellung hindert.

Zwischen der als Anschlag für den Prothesenkern 10 vorgesehenen Zunge 31 der Anschlagplatte 28 und der ihr gegenüberstehenden Fläche 13 des Kerns 10 kann sich - wie in Fig. 7 angedeutet - ein Freiraum von wenigen Millimetern befinden. Dadurch kann sich der Kern 10 in der von den seitlichen Abschnitten 8, 9 des Kragens 6 gebildeten Führung um eine gewisse Strecke in AP-Richtung bewegen (siehe die Beschreibung zu Fig. 4). Wenn dieses Bewegungsspiel nicht gewünscht wird, reduziert man den Freiraum zwischen der Fläche 13 und dem Anschlag 14 auf Null, wie es in Fig. 6 vorausgesetzt ist.

Für die dritte Ausführungsform gemäß Fig. 8 bis 12 gilt die obige Beschreibung zu Fig. 1 bis 3 mit Ausnahme der ventralen Anschlagvorrichtung.

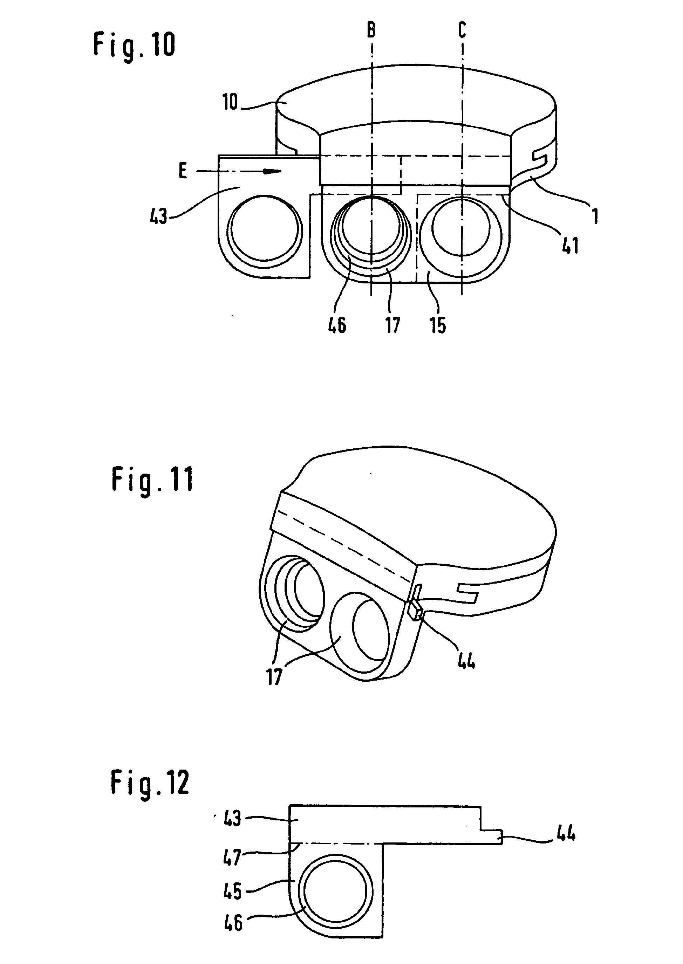

Entlang dem ventralen Rand der unteren Deckplatte 1 ist ein Schlitz 40 eingeschnitten, der in der in Fig. 10 linken Hälfte des Flanschs 15 vollständig von oben bis unten durchgeht und im rechten Teil des Flanschs 15 von der in Fig. 10 gestrichelt dargestellten Linie 41 begrenzt ist. Dem Schlitz 40 gegenüber ist in die Unterseite des Prothesenkerns 10 ein mit dem Schlitz 40 fluchtender Schlitz 42 eingeschnitten. Die beiden Schlitzen 40, 42 dienen zur Aufnahme einer Anschlagplatte 43 deren Umriß etwa den Begrenzungen des zu seiner Aufnahme bestimmten Raums gleicht, der durch die Schlitze 40, 42 gebildet ist. Wie Fig. 10 zeigt, kann sie nach dem Einsetzen des Prothesenkerns 10 von der Seite her in die Schlitze 40, 42 eingeschoben werden.

Die Anschlagplatte 43 kann am Ende mit einer Nase 44 versehen sein, die nach dem vollständigen Einschieben der Anschlagplatte 43 in die Schlitze 40, 42 auf der rechten Seite (siehe Fig. 11) herausschaut und zur Sicherung der Anschlagplatte umgebogen werden kann. Der Flansch 15 weist Schraubenlöcher 17 auf, die zur Befestigung der unteren Deckplatte 1 am zugehörigen Wirbelkörper dienen. Im linken Teil (siehe Fig. 10) durchquert das dortige Schraubenloch 17 den Schlitz 40, der sich dort über die gesamte Höhe des Flanschs 15 erstreckt, um den linken, breiten Flügel 45 der Anschlagplatte aufzunehmen. Dieser breite Flügel 45 der Anschlagplatte enthält ebenfalls ein Schraubenloch 46, daß dann, wenn die Anschlagplatte 43 eingeschoben ist (Fig. 9 und 11) mit dem dortigen Schraubenloch 17 fluchtet. Wenn sich in diesem Schraubenloch eine Befestigungsschraube befindet, kann die Anschlagplatte 43 nicht aus ihrer Sicherungsstellung entweichen.

Das Schraubenloch 46 und die Nase 44 bilden voneinander unabhängig wirkende Sicherungseinrichtungen. Es ist deshalb nicht erforderlich, daß stets alle beide vorgesehen sind. Wenn das Schraubenloch 46 vorhanden ist, kann die Nase 44 fehlen. Wenn die Nase vorgesehen ist, können der breite Flügel 45 der Anschlagplatte unterhalb der Linie 47 sowie der entsprechende Teil des Schlitzes im Befestigungsflansch 15 fehlen. Es genügt dann eine Anschlagplatte, die oberhalb der strichpunktierten Linie 47 (Fig. 12) verläuft. Damit die Anschlagplatte in diesem Falle auch nicht nach rechts aus dem Aufnahmeschlitz 40, 42 entweichen kann, kann eine der Nase 44 entsprechende Sicherungsnase (nicht gezeigt) auch am linken Ende der Anschlagplatte 43 vorgesehen sein. Nach oben kann die Anschlagplatte 43 nicht entweichen, weil sie überdeckt wird von demjenigen Teil des Prothesenkerns 10, der den Schlitz 42 bildet.

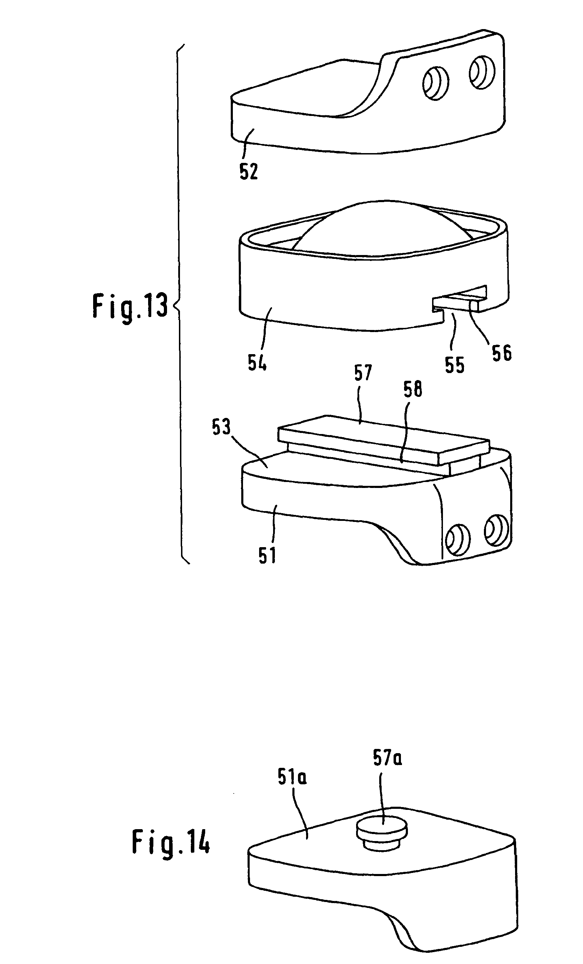

Die vierte Ausführungsform gemäß Fig. 13 und 14 veranschaulicht eine alternative Ausführungsmöglichkeit für den Sitz, der an der unteren Deckplatte 1 für den Prothesenkern 10 gebildet ist. In dieser Ausführungsform besteht die Prothese aus einer unteren Deckplatte 51 und einer oberen Deckplatte 52. Die untere Deckplatte weist eine obere, ebene Bodenfläche 53 auf, auf der der Prothesenkern 54 aufliegt. Während dieser Kern in den zuvor erläuterten Ausführungsformen an seinen Außenrändern geführt ist, besitzt er in der vierten Ausführungsform eine Ausnehmung 55 mit hinterschnittenen Innenrändern 56, die mit einem langgestreckten Vorsprung 57 der unteren Deckplatte mit entsprechend hinterschnittenen Rändern 58 zusammenwirken. Der Kern 54 wird dadurch - ebenso wie unter Bezugnahme auf das erste Ausführungsbeispiel erläutert - in AP-Richtung beweglich gegenüber der unteren Deckplatte 51 geführt. Außerdem ist er durch die zusammenwirkenden Hinterschnitte gegen ein Anheben von der unteren Deckplatte geschützt. Es sind nicht gezeigte Anschläge vorgesehen, die das dorsale und ventrale Herausrutschen des Prothesenkerns aus dem Plattenzwischenraum verhindern.

Die untere Deckplatte 51 kann durch die in Fig. 14 dargestellte untere Deckplatte 51a ersetzt werden, die sich von der unteren Deckplatte 31 dadurch unterscheidet, daß ihr Vorsprung 57a nicht langgestreckt, sondern in der Draufsicht kreisförmig begrenzt ausgeführt ist. Dies ermöglicht dem Prothesenkern 54, von dem angenommen wird, daß er in bezug auf eine vertikale Achse mit der oberen Deckplatte 52 drehverbunden ist, eine Rotation um den Vorsprung 57a, ohne die gewünschte AP-Bewegung zu behindern. Dies kann bei asphärischer Ausbildung der Gleitflächen zwischen Kern 54 und oberer Deckplatte 52 erwünscht sein.

## Patentansprüche

1. Zwischenwirbelprothese, insbesondere für die Halswirbelsäule, die im wesentliche aus einer ersten, mit einem ersten Wirbelkörper zu verbindenden Deckplatte (1, 51), einer zweiten, mit dem zweiten Wirbelkörper zu verbindenden Deckplatte (2, 52) und einem Prothesenkern (10, 54) besteht, der mit der zweiten Deckplatte (2, 52) ein Gelenk bildet und von einem Sitz der ersten Deckplatte (1, 51) gehalten ist, der als Führungseinrichtung (7, 11, 12, 56,57, 57a) ausgebildet ist, in die der Kern (10, 54) relativ zur ersten Deckplatte (1, 51) von der ventralen Seite her hineinbewegbar ist, **dadurch gekennzeichnet, daß** die erste Deckplatte (1, 51) an ihrem ventralen Rand eine Gleitführung (18, 29, 40) aufweist, in oder an welcher ein Anschlag (14, 31, 43) zwischen einer sperrenden Stellung und einer nicht sperrenden Stellung verschiebbar ist.

2. Prothese nach Anspruch 1 **dadurch gekennzeichnet, daß** eine Einrichtung (19, 33, 46, 44) zum Fixieren des Anschlags in der sperrenden Stellung vorgesehen ist.

3. Prothese nach Anspruch 1 oder 2 **dadurch gekennzeichnet, daß** die Gleitführung von einem quer zur Führungseinrichtung (7, 11, 12, 56,57, 57a) verlaufenden Führungsschlitz (18) gebildet ist, der den als Anschlagplatte (14) ausgebildeten Anschlag aufnimmt.

4. Prothese nach Anspruch 3 **dadurch gekennzeichnet, daß** der Führungsschlitz (18) eine quer zur Erstreckungsebene der ersten Deckplatte (1) verlaufende Führungsrichtung (D) hat und die in dieser Führungsrichtung (D) bewegbare Anschlagplatte (14) in ihrer sperrenden Stellung teilweise in dem Schlitz (18) gehalten ist und teilweise über die Bodenfläche (5) der ersten Deckplatte hinausragt.

5. Prothese nach Anspruch 4 **dadurch gekennzeichnet, daß** der Führungsschlitz (18) von wenigstens einem Schraubenloch (17) durchquert ist und die Anschlagplatte (14) ein in ihrer sperrenden Stellung damit fluchtendes Schraubenloch (19) aufweist.

6. Prothese nach Anspruch 4 **dadurch gekennzeichnet, daß** der Führungsschlitz (18) im ventralen Rand der ersten Deckplatte und einem daran angeordneten Befestigungsflansch (15) vorgesehen ist.

7. Prothese nach Anspruch 3 **dadurch gekennzeichnet, daß** der Führungsschlitz (40) eine parallel zur Erstreckungsebene der ersten Deckplatte (1) und quer zur AP-Richtung verlaufende Führungsrichtung (E) hat und die Anschlagplatte (43) in dieser Richtung in die sperrende Stellung bewegbar ist.

8. Prothese nach Anspruch 7 **dadurch gekennzeichnet, daß** der Prothesenkern (10) einen die im Führungsschlitz (40) enthaltende Anschlagplatte (43) überdeckenden Teil aufweist, der die Anschlagplatte (43) daran hindert, den Führungsschlitz (40) quer zu dessen Führungsrichtung (E) zu verlassen.

9. Prothese nach Anspruch 4 oder 8 **dadurch gekennzeichnet, daß** der Prothesenkern einen mit dem Führungsschlitz (49) fluchtenden, einen Teil der Anschlagplatte (43) aufnehmenden Schlitz (42) aufweist.

10. Prothese nach Anspruch 7 **dadurch gekennzeichnet, daß** der Führungsschlitz (40) von wenigstens einem Schraubenloch (17) durchquert ist und die Anschlagplatte (43) ein in ihrer sperrenden Stellung damit fluchtendes Schraubenloch (46) aufweist.

11. Prothese nach einem der Ansprüche 3 bis 10 **dadurch gekennzeichnet, daß** die Anschlagplatte (14, 43) eine in der Führungsrichtung (D,E) des Führungsschlitzes (18, 40) hinausragende, leicht biegbare Sicherungsnase (44) aufweist.

12. Prothese nach Anspruch 1 oder 2 **dadurch gekennzeichnet, daß** die Gleitführung für eine Anschlagplatte (28) von einer am ventralen Rand der ersten Deckplatte (1) quer zur AP-Richtung (A) verlaufenden Gleitführungsfläche und einem sich davon erhebenden Schwenkbolzen (29) gebildet ist, auf dem die Anschlagplatte (28) schwenkbar gelagert ist, und daß die Anschlagplatte (28) eine Zunge (31) aufweist, die in einer sperrenden Schwenkstellung der Anschlagplatte (28) über die Bodenfläche (5) der ersten Deckplatte hinausragt und in der nicht sperrenden Stellung unter die Bodenfläche (5) der ersten Deckplatte abgesenkt ist.

13. Prothese nach Anspruch 12 **dadurch gekennzeichnet, daß** in der Nachbarschaft des Schwenkbolzens (30) mindestens ein Schraubenloch (17) vorgesehen ist und die Anschlagplatte (30) in der sperrenden Stellung mit einem Flügel (30) über einen in dem Schraubenloch (17) befindlichen Schraubenkopf faßt.

14. Prothese nach einem der Ansprüche 1 bis 13 **dadurch gekennzeichnet, daß** die Gelenkfläche (22) des Prothesenkerns (10) im wesentlichen die gesamte erste Deckplatte (1) überdeckt.

15. Prothese nach Anspruch 14 **dadurch gekennzeichnet, daß** der Prothesenkern (10) relativ zur ersten Deckplatte (1) auch dann in AP-Richtung beweglich ist, wenn sich der Anschlag in der sperrenden Stellung befindet.

16. System von Zwischenwirbelprothesen, insbesondere für die Halswirbelsäule, die im wesentlichen aus einer ersten, mit einem ersten Wirbelkörper zu verbindenden Deckplatte (1, 51), einem zweiten, mit dem zweiten Wirbelkörper zu verbindenden Deckplatte (2, 52) und einem Prothesenkern (10, 54) bestehen, der von einem Sitz der ersten Deckplatte (1, 51) gehalten ist, der als Führungseinrichtung (7, 11, 12, 56, 57, 57a) ausgebildet ist, in die der Kern (10, 54) relativ zur ersten Deckplatte (1, 51) von der ventralen Seite her hineinbewegbar ist, und mit der zweiten Deckplatte (2, 52) ein Gelenk bildet, **dadurch gekennzeichnet, daß** das System neben Zwischenwirbelprothesentypen nach einem der Ansprüche 1 bis 15 solche Typen mit übereinstimmender Außenform umfaßt, die keine AP-Beweglichkeit zwischen dem Prothesenkern und der ersten Deckplatte aufweisen.

17. System nach Anspruch 16, **dadurch gekennzeichnet, daß** die Deckplatten (1, 2, 51, 52) der einander entsprechenden Typen mit bzw. ohne AP-Beweglichkeit übereinstimmen und der Kern (10, 54) unterschiedlich ist.

18. System nach Anspruch 16, **dadurch gekennzeichnet, daß** die zweite Deckplatte (2, 52) und der Prothesenkern (10, 54) der einander entsprechenden Typen mit bzw. ohne AP-Beweglichkeit übereinstimmen und die erste Deckplatte (1, 51) unterschiedlich ist.

19. System nach Anspruch 16, **dadurch gekennzeichnet, daß** alle drei Komponenten übereinstimmen und ein Anschlag (14, 31, 43), der die Beweglichkeit des Prothesenkerns ventral begrenzt, unterschiedlich ist.

## Claims

1. Intervertebral prosthesis, in particular for the cervical spine, consisting basically of a first cover plate (1, 51) to be connected to a first vertebral body, of a second cover plate (2, 52) to be connected to the second vertebral body, and of a prosthesis core (10, 54) which forms an articulation with the second cover plate (2, 52) and is held by a seat of the first cover plate (1, 51), which seat is designed as a guide device (7, 11, 12, 56, 57, 57a) into which the core (10, 54) can be moved from the ventral side relative to the first cover plate (1, 51), **characterized in that** the first cover plate (1, 51) has, on its ventral edge, a slide guide (18, 29, 40) in or on which a limit stop (14, 31, 43) is displaceable between a locking position and a nonlocking position.

2. Prosthesis according to Claim 1, **characterized in that** a device (19, 33, 46, 44) is provided for fixing the limit stop in the locking position.

3. Prosthesis according to Claim 1 or 2,
**characterized in that** the slide guide is formed by a guide slot (18) which extends transverse to the guide device (7, 11, 12, 56, 57, 57a) and which receives the limit stop designed as limit-stop plate (14).

4. Prosthesis according to Claim 3, **characterized in that** the guide slot (18) has a guide direction (D) extending transverse to the plane of extension of the first cover plate (1), and the limit-stop plate (14) which is movable in this guide direction (D) is, in its locking position, partially held in the slot (18) and partially extends above the bottom surface (5) of the first cover plate.

5. Prosthesis according to Claim 4, **characterized in that** the guide slot (18) has at least one screw hole (17) passing through it, and the limit-stop plate (14) has a screw hole (19) which is flush with the screw hole (17) when the limit-stop plate (14) is in its locking position.

6. Prosthesis according to Claim 4, **characterized in that** the guide slot (18) is provided in the ventral edge of the first cover plate and in a securing flange (15) arranged thereon.

7. Prosthesis according to Claim 3, **characterized in that** the guide slot (40) has a guide direction (E) extending parallel to the plane of extension of the first cover plate (1) and transverse to the AP direction, and the limit-stop plate (43) can be moved in this direction into the locking position.

8. Prosthesis according to Claim 7, **characterized in that** the prosthesis core (10) has a part which covers the limit-stop plate (43) held in the guide slot (40) and which prevents the limit-stop plate (43) from leaving the guide slot (40) transverse to the guide direction (E) thereof.

9. Prosthesis according to Claim 4 or 8,
**characterized in that** the prosthesis core has a slot (42) which is flush with the guide slot (49) and receives part of the limit-stop plate (43).

10. Prosthesis according to Claim 7, **characterized in that** the guide slot (40) has at least one screw hole (17) passing through it, and the limit-stop plate (43) has a screw hole (46) which is flush with the screw hole (17) when the limit-stop plate (43) is in its locking position.

11. Prosthesis according to one of Claims 3 to 10, **characterized in that** the limit-stop plate (14, 43) has an easily bendable fastening lug (44) projecting in the guide direction (D, E) of the guide slot (18, 40).

12. Prosthesis according to Claim 1 or 2,
**characterized in that** the slide guide for a limit-stop plate (28) is formed by a slide-guide surface extending transverse to the AP direction (A) on the ventral edge of the first cover plate (1), and by a pivot pin (29) which issues from the slide-guide surface and on which the limit-stop plate (28) is pivotably mounted, and **in that** the limit-stop plate (28) has a tongue (31) which, in a locking pivot position of the limit-stop plate (28), protrudes above the bottom surface (5) of the first cover plate, and, in the nonlocking position, is lowered below the bottom surface (5) of the first cover plate.

13. Prosthesis according to Claim 12, **characterized in that** at least one screw hole (17) is provided in the proximity of the pivot pin (30), and the limit- stop plate (30), in the locking position, engages with a wing (30) over a screw head located in the screw hole (17).

14. Prosthesis according to one of Claims 1 to 13, **characterized in that** the articulation surface (22) of the prosthesis core (10) covers substantially the entire first cover plate (1).

15. Prosthesis according to Claim 14, **characterized in that** the prosthesis core (10) is also movable relative to the first cover plate (1) in the AP direction when the limit stop is located in the locking position.

16. System of intervertebral prostheses, in particular for the cervical spine, consisting basically of a first cover plate (1, 51) to be connected to a first vertebral body, of a second cover plate (2, 52) to be connected to the second vertebral body, and of a prosthesis core (10, 54) which is held by a seat of the first cover plate, which seat is designed as a guide device (7, 11, 12, 56, 57, 57a) into which the core (10, 54) can be moved from the ventral side relative to the first cover plate (1, 51), and forms an articulation with the second cover plate (2, 52), **characterized in that** the system comprises, in addition to intervertebral prosthesis types according to one of Claims 1 to 15, types of matching outer form which have no AP mobility between the prosthesis core and the first cover plate.

17. System according to Claim 16, **characterized in that** the cover plates (1, 2, 51, 52) of the mutually corresponding types with or without AP mobility are the same and the core (10, 54) is different.

18. System according to Claim 16, **characterized in that** the second cover plate (2, 52) and the prosthesis core (10, 54) of the mutually corresponding types with or without AP mobility are the same and the first cover plate (1, 51) is different.

19. System according to Claim 16, **characterized in that** all three components are the same, and a limit stop (14, 31, 43), which limits the mobility of the prosthesis core in the ventral direction, is different.

## Revendications

1. Prothèse intervertébrale, en particulier pour la colonne cervicale, qui se compose essentiellement d'une première plaque de recouvrement (1, 51) à assembler à un premier corps de vertèbre, d'une seconde plaque de recouvrement (2, 52) à assembler au second corps de vertèbre, et d'un noyau de prothèse (10, 54), qui forme une articulation avec la seconde plaque de recouvrement (2, 52) et qui est maintenu par un siège de la première plaque de recouvrement (1, 51), qui est réalisé sous forme de système de guidage (7, 11, 12, 56, 57, 57a), dans lequel le noyau (10, 54) peut être déplacé par rapport à la première plaque de recouvrement (1, 51) vers l'intérieur depuis le côté ventral, **caractérisée en ce que** la première plaque de recouvrement (1, 51) présente sur son bord ventral une glissière (18, 29, 40), dans ou sur laquelle une butée (14, 31, 43) peut glisser entre une position bloquante et une position non bloquante.

2. Prothèse selon la revendication 1, **caractérisée en ce que** l'on prévoit un dispositif (19, 33, 46, 44) pour la fixation de la butée dans la position bloquante.

3. Prothèse selon la revendication 1 ou 2,
**caractérisée en ce que** la glissière est formée par une fente de guidage (18) s'étendant transversalement au dispositif de guidage (7, 11, 12, 56, 57, 57a), qui reçoit la butée réalisée sous forme de plaque de butée (14).

4. Prothèse selon la revendication 3, **caractérisée en ce que** la fente de guidage (18) a une direction de guidage (D) s'étendant transversalement au plan d'étendue de la première plaque de recouvrement (1) et la plaque de butée (14) déplaçable dans cette direction de guidage (D) est maintenue dans sa position bloquante en partie dans la fente (18) et sort en partie hors de la surface de fond (5) de la première plaque de recouvrement.

5. Prothèse selon la revendication 4, **caractérisée en ce que** la fente de guidage (18) est traversée par au moins un trou fileté (17) et la plaque de butée (14) présente un trou fileté (19) en affleurement avec celui-ci dans la position bloquante.

6. Prothèse selon la revendication 4, **caractérisée en ce que** la fente de guidage (18) est prévue dans le bord ventral de la première plaque de recouvrement et dans une bride de fixation (15) disposée sur celle-ci.

7. Prothèse selon la revendication 3, **caractérisée en ce que** la fente de guidage (40) a une direction de guidage (E) s'étendant parallèlement au plan d'étendue de la première plaque de recouvrement (1) et transversalement à la direction AP, et la plaque de butée (43) peut être déplacée dans cette direction dans la position bloquante.

8. Prothèse selon la revendication 7, **caractérisée en ce que** le noyau de prothèse (10) présente une partie recouvrant la plaque de butée (43) contenue dans la fente de guidage (40), laquelle empêche la plaque de butée (43) de quitter la fente de guidage (40) transversalement à sa direction de guidage (E).

9. Prothèse selon la revendication 4 ou 8,
**caractérisée en ce que** le noyau de prothèse présente une fente (42) en affleurement avec la fente de guidage (49), recevant une partie de la plaque de butée (43).

10. Prothèse selon la revendication 7, **caractérisée en ce que** la fente de guidage (40) est traversée par au moins un trou fileté (17) et la plaque de butée (43) présente un trou fileté (46) en affleurement avec celui-ci dans sa position bloquante.

11. Prothèse selon l'une quelconque des revendications 3 à 10, **caractérisée en ce que** la plaque de butée (14, 43) présente un nez de fixation (44) légèrement flexible, saillant dans la direction de guidage (D, E) de la fente de guidage (18, 40).

12. Prothèse selon la revendication 1 ou 2,
**caractérisée en ce que** la glissière pour une plaque de butée (28) est formée d'une surface de glissière s'étendant au niveau du bord ventral de la première plaque de recouvrement (1) transversalement à la direction AP (A) et d'un boulon pivotant (29) saillant depuis celle-ci, sur lequel la plaque de butée (28) est montée à pivotement, et **en ce que** la plaque de butée (28) présente une langue (31) qui fait saillie dans une position de pivotement bloquante de la plaque de butée (28) au-delà de la surface de fond (5) de la première plaque de recouvrement et est enfoncée dans la position non bloquante sous la surface de fond (5) de la première plaque de recouvrement.

13. Prothèse selon la revendication 12, **caractérisée en ce qu'**au moins un trou fileté (17) est prévu à proximité du boulon pivotant (30) et retient la plaque de butée (30) dans la position bloquante avec une aile (30) par le biais d'une tête filetée située dans le trou fileté (17).

14. Prothèse selon l'une quelconque des revendications 1 à 13, **caractérisée en ce que** la surface d'articulation (22) du noyau de prothèse (10) recouvre essentiellement toute la première plaque de recouvrement (1).

15. Prothèse selon la revendication 14, **caractérisée en ce que** le noyau de prothèse (10) peut également être déplacé par rapport à la première plaque de recouvrement (1) dans la direction AP, lorsque la butée se trouve dans la position bloquante.

16. Système de prothèses intervertébrales, en particulier pour la colonne cervicale, qui se composent essentiellement d'une première plaque de recouvrement (1, 51) à assembler à un premier corps de vertèbre, d'une seconde plaque de recouvrement (2, 52) à assembler au second corps de vertèbre, et d'un noyau de prothèse (10, 54), qui est maintenu par un siège de la première plaque de recouvrement (1, 51), qui est réalisé sous forme de système de guidage (7, 11, 12, 56, 57, 57a), dans lequel le noyau (10, 54) peut être déplacé par rapport à la première plaque de recouvrement (1, 51) vers l'intérieur depuis le côté ventral, et qui forme une articulation avec la seconde plaque de recouvrement (2, 52)
**caractérisé en ce que** le système comprend, en plus des types de prothèses intervertébrales selon l'une quelconque des revendications 1 à 15, des types ayant des formes extérieures complémentaires, qui ne présentent aucune mobilité AP entre le noyau de prothèse et la première plaque de recouvrement.

17. Système selon la revendication 16, **caractérisé en ce que** les plaques de recouvrement (1, 2, 51, 52) des types à correspondance mutuelle avec ou sans mobilité AP coïncident et le noyau (10, 54) est différent.

18. Système selon la revendication 16, **caractérisé en ce que** la deuxième plaque de recouvrement (2, 52) et le noyau de prothèse (10, 54) des types à correspondance mutuelle avec ou sans mobilité AP coïncident et la première plaque de recouvrement (1, 51) est différente.

19. Système selon la revendication 16, **caractérisé en ce que** les trois composants coïncident et une butée (14, 31, 43), qui limite ventralement la mobilité du noyau de prothèse, est différente.
